# EUROPÄISCHE PATENTSCHRIFT

(11) **EP 2 218 420 B1**
(45) Veröffentlichungstag und Bekanntmachung des Hinweises auf die Patenterteilung: **16.11.2011**
(21) Anmeldenummer: 09002027.2
(22) Anmeldetag: 13.02.2009
(51) Int. Cl.: A61B 19/00, A61L 2/18

(54) **Verfahren zur Aufbereitung medizinischer und/oder chirurgischer Instrumente und Apparate**
Method for preparing medical and/or surgical instruments and devices
Procédé de préparation d'instruments et appareils médicaux et/ou chirurgicaux

(43) Veröffentlichungstag der Anmeldung: 18.08.2010
(73) Patentinhaber: Chemische Fabrik Dr. Weigert GmbH & Co. KG, 20539 Hamburg (DE)
(72) Erfinder: Gautheron, Luc, 60750 Choisy-Bacau (FR)
(74) Vertreter: Glawe, Delfs, Moll

(56) Entgegenhaltungen:
- EP-A- 1 709 978
- WO-A-2007/000639
- DE-A1- 10 203 225

## Beschreibung

Die Erfindung betrifft ein Verfahren zur Aufbereitung medizinischer und/oder chirurgischer Instrumente und Apparate.

Chirurgische Instrumente durchlaufen einen sogenannten Instrumentenzyklus. Nach Gebrauch im OP werden sie vom Chirurg bzw. OP-Personal in einem Behälter abgelegt und der Aufbereitung zugeführt. Gebräuchlich ist insbesondere in Frankreich die sogenannte Prädesinfektion als Nassentsorgungsmethode, bei denen die Instrumente in einen großen Tauchbehälter gefüllt mit Desinfektionslösung abgelegt werden. Diese Tauchbehälter enthalten häufig 50 l Desinfektionslösung.

Die entsorgten Instrumente werden zu einer zentralen Instrumentenaufbereitung gebracht. Die Instrumentenaufbereitung erfolgt in Krankenhäusern inzwischen weitestgehend zentral, da nach dem Stand der Technik und relevanter Empfehlungen zur Krankenhaushygiene eine dezentrale Aufbereitung nicht empfohlen wird. So ist insbesondere vorgesehen, dass die zentrale Aufbereitung eine sogenannte unreine und eine reine Seite aufweisen muss, für den Zugang zur reinen Seite sind beispielsweise Schleusen vorgesehen, durch die die reine Seite betreten werden kann. Dies ist wirtschaftlich nur in einer zentralen Aufbereitung darstellbar.

In der zentralen Aufbereitung werden die entsorgten Instrumente der maschinellen Reinigung und Desinfektion zugeführt. Nach dem Reinigen und Desinfizieren werden sie auf der reinen Seite entnommen und in der Regel zu gängigen OP-Sets verpackt, die beispielsweise die erforderlichen Instrumente für eine bestimmte Operation enthalten. Diese Sets werden dann in einem weiteren Schritt dampfsterilisiert (134°C) und für die Verwendung gelagert.

Von den entsorgten Instrumenten geht für das handhabende Personal und ggf. auch Umwelt oder Patienten eine Kontaminationsgefahr aus. Die nasse Entsorgung soll diese Kontaminationsgefahr minimieren und bereits eine Vordesinfektion gewährleisten, sie leistet dies jedoch häufig nur unzureichend und kann insbesondere abhängig von dem gewählten Desinfektionsmittel eine sogenannte Fixierung von Verunreinigungen an den benutzten Instrumenten verursachen, beispielsweise durch chemische Denaturierung von Proteinen. Ferner bedeutet es einen hohen Aufwand, Tauchbäder mit 50 1 Desinfektionslösung und darin gelagerten Instrumenten zu einer zentralen Instrumentenaufbereitung zu fahren.

In den Absatz der WO 2007/000639, auf Seite 1, Zeilen 10-23, der sich auf Stand der Technik bezieht wird ein Verfahren beschrieben, wonach die gebrauchten chirurgischen Instrumente für Desinfektion in Tauchbehälter mit Desinfektionsmittel hineingelegt werden und weiterhin mittels Instrumentenspülmaschine behandelt werden. Am Ende des Waschens werden schon getrocknete chirurgische Instrumente eingepackt und zur Sterilisationsbehandlung verschickt.

Der Erfindung liegt die Aufgabe zugrunde, ein Verfahren der eingangs genannten Art zu schaffen, das den Gesamtvorgang der Aufbereitung in dem sogenannten Instrumentenzyklus verbessert. Das erfindungsgemäße Verfahren weist folgende Schritte auf:
a) Einlegen der gebrauchten Instrumente und/oder Apparate in eine Instrumentenspülmaschine, vorzugsweise in eine Eintank-Instrumentenspülmaschine;
b) Durchführen einer maschinellen Prädekontamination;
c) Entnehmen der Instrumente und/oder Apparate aus der Instrumentenspülmaschine;
d) Überführen der Instrumente und/oder Apparate zu einer zentralen Instrumentenaufbereitungsstation;
e) Durchführen einer vollständigen Reinigung, Desinfektion und Sterilisation der Instrumente und/oder Apparate in der zentralen Instrumentenaufbereitungsstation zur Wiederherstellung eines gebrauchsfertigen Zustands der Instrumente und/oder Apparate.

Zunächst seien einige im Rahmen der Erfindung verwendete Begriffe erläutert.

Medizinische und/oder chirurgische Instrumente und Apparate sind sämtliche in den medizinischen und Krankenhausbereich eingesetzten Geräte sowie Teile davon, die einer maschinellen Reinigung und Desinfektion grundsätzlich zugänglich sind. "Maschinell" bedeutet, dass das Verfahren automatisch abläuft und im Zuge der Prädekontamination einschließlich Prädesinfektion kein menschlicher Eingriff erforderlich ist. Insbesondere kann erfindungsgemäß in einer üblichen Spülmaschine für chirurgische Instrumente maschinell vorgereinigt werden.

Der Begriff Prädekontamination bezeichnet eine Vorreinigung einschließlich Prädesinfektion, bei der einerseits wesentliche Teile der Verschmutzung bereits entfernt werden und andererseits an den Instrumenten anhaftende Mikroorganismen (insbesondere Bakterien, Viren und Pilze, ggf. Prionen) zumindest soweit reduziert werden, dass noch anhaftende Restmengen von Mikroorganismen keine oder nur eine geringe Gefährdung für das Personal darstellen, das diese Instrumente zu der zentralen Instrumentenaufbereitungsstation überführt. Somit verfolgt diese Prädekontamination den Zweck, die Population von Mikroorganismen auf den Instrumenten deutlich zu verringern und so zum einen die endgültige Reinigung und Desinfektion in der zentralen Instrumentenaufbereitungsstation zu erleichtern und zum anderen das die vorgereinigten Instrumente handhabende Personal vor Kontamination zu schützen. Auch eine Verunreinigung der Umwelt mit Mikroorganismen kann vermieden oder zumindest verringert werden.

Im Rahmen der erfindungsgemäßen Prädekontamination können vorzugsweise chemische Desinfektionsmittel verwendet werden, die die Anforderungen der DIN-EN 1040:2005 (Basistest Bakterizidie) und/oder DIN-EN 1275:2005 (Fungizidie und Levurozidie) erfüllen. Bevorzugt erfüllen die Mittel beide genannten Normen.

Weiter bevorzugt erfüllen die Desinfektionsmittel zusätzlich die Anforderungen der DIN-EN 13727:2003 (Quantitativer Suspensionsversuch zur Prüfung der bakteriziden Wirkung chemischer Desinfektionsmittel für Instrumente im humanmedizinischen Bereich) und/oder DIN-EN 13624:2003 (Quantitativer Suspensionsversuch zur Prüfung der fungiziden und levuroziden Wirkung chemischer Desinfektionsmittel für Instrumente im humanmedizinischen Bereich) erfüllen. Bevorzugt erfüllen die Mittel beide genannten Normen, insgesamt also alle vier genannten Normen. Zur Anwendung der genannten Normen und weitere Begriffsbestimmungen wird auf die DIN-EN 14885:2006 (Anwendung Europäischer Normen für chemische Desinfektionsmittel und Antiseptika) verwiesen.

Weiter bevorzugt erfüllen die Desinfektionsmittel zusätzlich die Anforderungen hinsichtlich der begrenzten Viruzidie, wie sie definiert sind in der Empfehlung "Prüfung und Deklaration der Wirksamkeit von Desinfektionsmitteln gegen Viren" des Arbeitskreises Viruzidie beim Robert Koch-Institut (RKI) sowie des Fachausschusses "Virusdesinfektion" der Deutschen Vereinigung zur Bekämpfung der Viruskrankheiten (DVV) und der Desinfektionsmittelkommission der Deutschen Gesellschaft für Hygiene und Mikrobiologie (DGHM), Bundesgesundheitsbl - Gesundheitsforsch - Gesundheitsschutz 2004, 47:62-66.

Diese umfasst die Wirksamkeit gegen die über Blut übertragbaren Viren Hepatitis B, Hepatitis C und HI-Virus

Die maschinelle Prädekontamination kann vorzugsweise in einer gebräuchlichen Instrumentenspülmaschine durchgeführt werden.

Nach Abschluss der Prädekontamination können die Instrumente bzw. Apparate aus der Spülmaschine entnommen und zu der zentralen Instrumentenaufbereitungsstation überführt werden. Von den vorgereinigten und prädesinfizierten Instrumenten geht, wie geschildert, keine oder nur eine geringe Kontaminationsgefahr für handhabendes Personal und Umwelt aus. Die Instrumente können offen transportiert werden, es ist kein aufwendiger nasser Transport bspw. eingelegt in große Tanks mit Desinfektionslösung erforderlich. Das erfindungsgemäße Verfahren hat den weiteren Vorteil, dass durch die maschinelle Prädekontamination, anders als beim Einlegen in Desinfektionslösungen, keine sogenannte Proteinfixierung von Verunreinigungen an den Instrumenten stattfindet, die die anschließende zentrale Instrumentenaufbereitung signifikant erschweren kann.

In der zentralen Instrumentenaufbereitungsstation wird eine vollständige Reinigung, Desinfektion und anschließende Sterilisation der Instrumente und Apparate durchgeführt. Ergebnis dieser Aufbereitung sind gebrauchsfertig zur erneuten Verwendung hergerichtete Instrumente, bspw. verpackte OP-Sets. Erfindungsgemäß kann die Sterilisation auch getrennt von der vollständigen Reinigung und Desinfektion in der zentralen Instrumentenaufbereitungsstation stattfinden. Bspw. können die gereinigten und desinfizierten Instrumente verpackt und dann ggf. räumlich (und wahlweise zeitlich) getrennt von der Aufbereitung der zentralen Instrumentenaufbereitungsstation sterilisiert, bspw. dampfsterilisiert werden.

Das erfindungsgemäße Verfahren kombiniert somit eine dezentrale Prädekontamination bzw. Prädesinfektion (vorzugsweise unmittelbar im Operationsbereich bzw. Operationssaal) mit einer zentralen Instrumentenaufbereitung zur endgültigen Reinigung, Desinfektion und Sterilisierung oder Vorbereitung auf die anschließende Sterilisierung. Die maschinelle Prädekontamination und Prädesinfektion erlaubt eine problemlose Handhabung der Instrumente auf dem Weg zur zentralen Aufbereitung und erlaubt es, diese zentrale Aufbereitung ggf. unter weniger drastischen Bedingungen durchzuführen, da wesentliche Teile der Verschmutzungen bzw. Populationen von Mikroorganismen bereits entfernt sind bzw. verhindert wurde, dass diese sich im Zeitraum zwischen der Benutzung und der Durchführung der zentralen Instrumentenaufbereitung bspw. durch chemische Prozesse an den Instrumenten fixieren.

Die Instrumentenspülmaschine zur Durchführung der maschinellen Prädekontamination ist bevorzugt im Operationsbereich angeordnet. Dies kann insbesondere der Operationssaal oder ein unmittelbar angrenzender Nebenraum sein, so dass die Instrumente unmittelbar nach ihrer Verwendung zur Durchführung der Prädekontamination in diese gewissermaßen lokale Instrumentenspülmaschine eingelegt werden können. Erfindungsgemäß kann die maschinelle Prädekontamination eine Reinigung umfassen mit einem tensidhaltigen Reinigungsmittel, das anwendungsfertig verdünnt in wässriger Lösung einen pH-Wert von wenigstens 10,5 aufweist. Die maschinelle Prädekontamination kann insbesondere folgende Schritte umfassen:
a) Vorspülen;
b) Reinigen mit einem tensidhaltigen Reinigungsmittel, das anwendungsfertig verdünnt in wässriger Lösung einen pH-Wert von wenigstens 10,5 aufweist;
c) Neutralisieren und/oder Spülen.

Das Vorspülen kann bspw. ein Vorspülen lediglich mit Wasser sein. Die Reinigung mit dem vorstehend definierten Reinigungsmittel bewirkt gleichzeitig eine hinreichende Prädesinfektion, bei der Mikroorganismen, insbesondere Bakterien, Viren und/oder Pilze, ggf. auch Prionen, hinreichend in ihrer Population vermindert werden, so dass die Instrumente anschließend gefahrlos der zentralen Instrumentenaufbereitung zugeführt werden können.

Der Begriff Neutralisieren bezeichnet ein Neutralisieren etwaiger Restalkalität auf den Instrumenten mit einer sauren Lösung. Alternativ oder zusätzlich kann wässrig nachgespült werden.

Ein bevorzugter Inhaltsstoff des tensidhaltigen Reinigungsmittels sind Alkalihydroxide, insbesondere Kaliumhydroxid. Bevorzugte Alkalihydroxidgehalte in der anwendungsfertig verdünnten wässrigen Lösung sind 200 bis 10.000 ppm, weiter vorzugsweise 200 bis 5.000 ppm, weiter vorzugsweise 200 bis 2.000 ppm. Die Verwendung von Kaliumhydroxid erleichert das Bereitstellen des Reinigungsmittels in Form eines (vor der Anwendung zu verdünnenden) Konzentrats, da Kaliumhydroxidlösungen bei niedrigen Temperaturen weniger zur Auskristallisation neigen als Natriumhydroxidlösungen. Bei den vorstehenden Konzentrationsangaben bezieht sich die Angabe ppm auf Gewichtsteile.

Die Einwirkzeit des Reinigungsmittels kann bevorzugt zwischen 1 und 30 min, weiter vorzugsweise 2 und 20 min, weiter vorzugsweise 3 und 15 min, weiter vorzugsweise 5 und 10 min liegen.

Die Prädekontamination bzw. Prädesinfektion kann bevorzugt bei einer Temperatur zwischen Raumtemperatur und 93°C, weiter vorzugsweise 40 und 93°C, weiter vorzugsweise 50 und 80°C, weiter vorzugsweise 50 und 60°C durchgeführt werden.

Bspw. kann eine Reinigung bei 55°C mit einer Einwirkdauer von 5 min im Regelfall eine hinreichende Bakterizidie, Levurozidie, begrenzte Viruzidie (Wirksamkeit gegen behüllte Viren) und Prionendestabilisierung sicherstellen. Destabilisieren von Prionen bedeutet, dass ggf. auf der Instrumentenoberfläche anhaftendes infektiöses Prionenmaterial zumindest partiell destabilisiert wird. Bei einer Destabilisierung liegt die pathogene Konformation des Prionenmoleküls nicht mehr vor.

Verlängert man die Reinigungsdauer auf bspw. etwa 10 min, kommt es in der Regel zu einer Prionen-Destabilisierung, - Inaktivierung oder -Dekontamination.

Die Destabilisierung wird durch einen in vitro-Test bestätigt, bei welchem kontaminierte Edelstahloberflächen einem Behandlungsprozess unterzogen werden und anschließend sowohl die Edelstahloberfläche als auch die Reinigungsflüssigkeit biochemisch, z.B. mittels Western Blot, untersucht werden. Werden weder auf der Edelstahloberfläche noch in der Reinigungslösung mittels biochemischer Prüfung Prionen nachgewiesen, wird diese Wirksamkeit als Prionen destabilisierend bezeichnet.

Die Prionen-Inaktivierung wird mittels quantitativen Suspensionstests eines mit Prionen kontaminierten Hirnhomogenates, welches mit der zu prüfenden Flüssigkeit gemischt wird, geprüft. Bei einer intracerebralen Impfung des behandelten Prion-Hirnhomogenates in gesunde Versuchstiere muss eine Prionabreicherung um mindestens 4 log-Stufen erfolgen, dann darf eine Prionen-Inaktivierung ausgelobt werden.

Die Prionen-Dekontamination wird in einem quantitativen Carrier-Test mit Edelstahlstiften geprüft, die mit dem mit Prionen infizierten Hirnhomogenat kontaminiert werden. Nach Behandlung mit der zu prüfenden Reinigerlösung werden die Stifte intracerebral in gesunde Versuchstiere implantiert. Eine Prionabreicherung um mindestens 4 log-Stufen führt zur Wirksamkeitsstufe der Prionen-Dekontamination unter der Voraussetzung, dass auch der vorgenannte Test zur Prüfung der Prionen-Inaktivierung bestanden wurde.

Die genannten Testverfahren sind detailliert erläutert in J. Bertram, M. Mielke, M.Beekes, K. Lemmer, M. Baier, G. Pauli, Inaktivierung und Entfernung von Prionen bei der Aufbereitung von Medizinprodukten, Bundesgesundheitsbl - Gesundheitsforsch - Gesundheitsschutz 2004, 47:36-40.

Das tensidhaltige Reinigungsmittel kann anwendungsfertig verdünnt in wässriger Lösung höhere pH-Werte aufweisen, bspw. wenigstens 11, 11,5, 12 oder 12,5. pH 11 ist erfindungsgemäß eine besonders bevorzugte Untergrenze für eine gute prädekontaminierende Wirkung.

Das Reinigungsmittel kann kationische, nichtionische und/oder amphotere Tenside enthalten. Es hat sich gezeigt, dass eine Kombination von Alkalität und wenigstens zwei Tensiden aus der genannten Gruppe eine besonders gute Wirkung gegen Bakterien, Viren und Pilze aufweist. Ein weiterer Bestandteil des Reinigungsmittels können Alkanolamine sein.

Der Gehalt bei kationischen Tensiden in der anwendungsfertig verdünnten Lösung liegt vorzugsweise zwischen 15 und 500 ppm, weiter vorzugsweise 15 und 100 ppm, weiter vorzugsweise 15 und 50 ppm. Ein bevorzugter Gehalt an nichtionischen Tensiden ist 15 bis 500 ppm, vorzugsweise 15 bis 200 ppm, weiter vorzugsweise 25 bis 100 ppm. Ein bevorzugter Gehalt an amphoteren Tensiden ist 50 bis 1.000 ppm, weiter vorzugweise 100 bis 500 ppm, weiter vorzugsweise 150 bis 300 ppm.

Als kationische Tenside sind quarternäre Ammoniumverbindungen (QAV) besonders bevorzugt. Diese wirken im Rahmen der erfindungsgemäßen Kombination auch bei sehr niedrigen Anwendungskonzentrationen mikrobiozid.

Durch Zusatz von Tensiden zu der hochalkalischen Reinigerlösung kann die Oberflächen- und Grenzflächenspannung deutlich reduziert werden. Grundsätzlich sind nichtionische Tenside wie bspw. Fettalkohole am besten zur Verminderung der Oberflächenspannung einer wässrigen Lösung geeignet. Sie haben den zusätzlichen Vorteil, dass sie wenig schäumen und somit die unerwünschte Schaumbildung bei der maschinellen Prädekontamination medizinischer Instrumente verhindern oder vermindern. Eine Schaumbildung kann insbesondere den Umwälzpumpendruck in der Spülmaschine reduzieren, die Reinigung bspw. englumiger Schläuche von Endoskopen oder dergleichen beeinträchtigen.

Die anwendungsfertig verdünnte Reinigerlösung weist vorzugsweise eine Oberflächenspannung von weniger als 50mN/m, vorzugsweise weniger als 40 mN/m, weiter vorzugsweise weniger als 35 mN/m auf. Die Oberflächenspannung wird bestimmt nach der sogenannten Bügel-Ring-Methode nach DIN 53993.

Besonders bevorzugt ist im Rahmen des erfindungsgemäßen Verfahrens bzw. der dabei verwendeten Reinigerlösung die Kombination von Alkalität zur Einstellung eines pH-Werts von wenigstens 11 in anwendungsfertig verdünnter Reinigerlösung mit quarternären Ammoniumverbindungen (QAV) und amphoteren Tensiden (bspw. Alkylaminocarbonsäuresalze). Amphotere Tenside dienen zusätzlich als Löslichkeitsvermittler für QAV. Bevorzugt beträgt das Gewichtsverhältnis von amphoteren Tensiden zu QAV in der Reinigerlösung 2,3:1 bis 5:1, weiter vorzugsweise 2,3:1 bis 4:1, besonders bevorzugt 2,3:1 bis 3:1. Die Konzentration der QAV in der verdünnten Reinigerlösung liegt vorzugsweise zwischen 15 und 100 ppm, weiter vorzugsweise 15 und 50 ppm, weiter vorzugsweise 15 und 30 ppm (jeweils Gewichtsteile).

Typischerweise wird die anwendungsfertig verdünnte Reinigerlösung aus einem Konzentrat mit Wasser angesetzt, typische Einsatzkonzentrationen eines solchen Konzentrats können bspw. 0,2 bis 5 Gew.-%, vorzugsweise 0,2 bis 2 Gew.-%, weiter vorzugsweise 0,5 bis 1 Gew.-% sein.

Im Rahmen der Erfindung kann das Überführen der vorgereinigten Instrumente bzw. Apparate zu der zentralen Instrumentenaufbereitungsstation trocken erfolgen. Trocken bedeutet in diesem Zusammenhang, dass die Instrumente in geeigneten offenen oder verschlossenen Behältern transportiert werden und nicht nass eingelegt in einer Desinfektionslösung transportiert werden müssen.

Ausführungsbeispiele der Erfindung werden im Folgenden beschrieben. Die Figur zeigt schematisch einen sogenannten Instrumentenkreislauf im Krankenhaus, bei dem das erfindungsgemäße Verfahren zum Einsatz kommt.

### Beispiel 1

Ein Reinigungsmittelkonzentrat wird gemäß der nachfolgenden Tabelle 1 zubereitet. Die Mengen der einzusetzenden Ausgangsstoffe sind in Gewichtsteilen angegeben. (Rest auf 100 Gewichtsteile Wasser). i-Octylaminodipropionat ist ein amphoteres Tensid.

| | |
|---|---|
| Alkali-Polymerphosphat | 21,39 |
| Kaliumhydroxid | 9,90 |
| i-Octylaminodipropionat (Na-Salz) | 2,40 |
| n-Tensid, C10/12-Alkyl-EO/PO-Addukt | 0,50 |
| Dialkyl-dimethyl-Ammoniumsalz¹ (QAV) | 0,25 |
| Alkaliwasserglas | 27,90 |

| | |
|---|---|
| ¹Kationisches Tensid | |

### Beispiel 2

In diesem Beispiel wird die Wirksamkeit der Formulierung des Beispiels 1 bei einer maschinellen Prädekontamination geprüft.

Die desinfizierenden Eigenschaften eines Produktes zur maschinellen Vorreinigung mit desinfizierender Wirkung dienen vor allem dem Personalschutz der durchführenden Personen. Der Patientenschutz spielt hier eine untergeordnete Rolle, da die Medizinprodukte anschließend noch einer Aufbereitung (mindestens Reinigung und Desinfektion) zugeführt werden. Erreger, die durch das kontaminierte, verschmutzte Material z.B. in die Desinfektionslösung eingebracht werden, müssen auch unter Schmutzbelastung schnell abgetötet werden, sodass eine Infektion des Personals vermieden wird. Das für die Vorreinigung mit desinfizierender Wirkung zu berücksichtigende Erregerspektrum umfasst vor allem Bakterien und Hefepilze sowie behüllte Viren (Hepatitis B, HI-Viren). Demzufolge ist eine bakterizide, levurozide und begrenzt viruzide Wirksamkeit (Wirksamkeit gegen HBV, HIV, HCV) erwünscht.

### Bakterizidie

Zum Nachweis der Bakterizidie wird die DIN-EN 13727 (Test unter Anwendungsbedingungen) verwendet. Es handelt sich um einen quantitativen Suspensionsversuch zur Ermittlung der wirksamen Konzentration unter den im Test definierten Bedingungen. Für den Nachweis der bakteriziden Wirksamkeit für die Prädekontamination mit desinfizierender Wirkung werden die Untersuchungen unter sogenannter "hoher Belastung" durchgeführt, da es sich um eine kombinierte Reinigung und Desinfektion handelt.

Eine aus dem Konzentrat gemäß Beispiel 1 hergestellte wässrige Reinigerlösung wurde nach der DIN-EN 13727 untersucht. Es wurden die Testkeime Staphylococcus aureus, Pseudomonas aeruginosa und Enterococcus hirae zur Prüfung der bakteriziden Wirkung verwendet. Die erforderliche Keimreduktion von 5 log Stufen wurde unter folgenden Bedingungen erreicht:

| | |
|---|---|
| - Konzentration des Bspl. 1 in Wasser: | 1 Gew.-% |
| - Temperatur: | 55°C |
| - Kontaktzeit: | 5 min |

### Levurozidie

Zum Nachweis der Levurozidie (Wirksamkeit gegen Hefepilze, Testkeim: Candida albicans) wird die Norm DIN-EN 13624 (Test unter Anwendungsbedingungen) verwendet. Es handelt sich um einen quantitativen Suspensionsversuch zur Ermittliung der wirksamen Konzentration unter den im Test definierten Bedingungen. Für den Nachweis der levuroziden Wirksamkeit die Prädekontamination mit desinfizierender Wirkung werden die Untersuchungen unter sogenannter "hoher Belastung" durchgeführt, da es sich um eine kombinierte Reinigung und Desinfektion handelt.

Eine aus dem Konzentrat gemäß Beispiel 1 hergestellte wässrige Reinigerlösung wurde nach der DIN-EN 13624 untersucht. Es wurde der Testkeim Candida albicans zur Prüfung der levuroziden Wirkung verwendet. Die erforderliche Keimreduktion von 4 log Stufen wurde unter folgenden Bedingungen erreicht:

| | |
|---|---|
| - Konzentration des Bspl. 1 in Wasser: | 1 Gew.-% |
| - Temperatur: | 55°C |
| - Kontaktzeit: | 5 min |

### Viruzidie

Zum Nachweis der viruziden Wirksamkeit wird die Prüfung nach den Methoden der Deutschen Vereinigung zur Bekämpfung der Viruskrankheiten (DVV) und des Robert Koch-Institutes (RKI) durchgeführt. Gemäß der RKI-Empfehlung "Prüfung und Deklaration der Wirksamkeit von Desinfektionsmitteln gegen Viren" vom Januar 2004 (Bundesgesundheitsbl - Gesundheitsforsch - Gesundheitsschutz 2004, 47:62-66) wird die Wirkung gegen behüllte Viren (begrenzt viruzid) und die Wirkung gegen unbehüllte und behüllte Viren (viruzid) unterschieden. Zum Nachweis der begrenzten Viruzidie ist die Wirkung gegen die Testviren BVDV (Bovine Viral Diarrea Virus) und Vaccinia Virus zu erbringen. Die Wirksamkeit gegen diese Viren schließt die Wirksamkeit gegen HBV, HCV und HIV mit ein.

Eine aus dem Konzentrat gemäß Beispiel 1 hergestellte wässrige Reinigerlösung wurde nach Methode DVV/RKI untersucht. Die erforderliche Reduktion des Virustiters von 4 log Stufen (bei Eiweißbelastung "hoch") wurde unter folgenden Bedingungen erreicht:

| | |
|---|---|
| - Konzentration des Bspl. 1 in Wasser: | 1 Gew.-% |
| - Temperatur: | 20°C |
| - Kontaktzeit: | 5 min |

Gegen den Testkeim Vaccinia Virus wurde eine entsprechende Reduktion auch schon bei einer Konzentration des Reinigerkonzentrats von 0,5 Gew.-% erreicht.

Folgende Anwendungsbedingen gewährleisten somit bei diesem Ausführungsbeispiel eine umfassende Prädekontamination:

| | |
|---|---|
| - Konzentration des Bspl. 1 in Wasser: | 1 Gew.-% |
| - Temperatur: | 55°C |
| - Kontaktzeit: | 5 min |

### Beispiel 3

Dieses Beispiel beschreibt einen vollständigen Instrumentenzyklus unter Einschluss eines erfindungsgemäßen Verfahrens.

Im OP-Saal 1 genutzte chirurgische Instrumente wurden nach Gebrauch in eine übliche Eintankinstrumentenspülmaschine 2 eingelegt, die sich im Operationssaal befindet. Die Prädekontamination umfasst folgende Schritte:
- Vorspülung mit Wasser 1 min,
- Reinigung und chemothermische Desinfektion mit einer 1%igen wässrigen Lösung des Reinigers gemäß Beispiel 1, Einwirkzeit 5 min, Einwirktemperatur 55°C,
- Zwischenspülen mit Wasser bei Raumtemperatur, 1 min,
- Schlussspülung mit voll entsalztem Wasser, 1 min,

Die so vorgereinigten Instrumente werden zur zentralen Instrumentenaufbereitung 3 transportiert. Von der sog. unreinen Seite der zentralen Instrumentenaufbereitung werden sie einer im Stand der Technik bekannten Instrumentenaufarbeitung unterzogen. Die Reinigung und Desinfektion erfolgt in einer Instrumentenspülmaschine und umfasst folgende Schritte:
- Kaltwasservorspülung, 3min,
- Reinigung mit 0,5%iger wässriger Lösung von neodisher mediclean forte® (Alkalischer Reiniger der Chemischen Fabrik Dr. Weigert GmbH & Co. KG), 55°C, 10 min,
- Zwischenspülen mit Wasser bei Raumtemperatur, 1 min,
- Thermodesinfektion mit VE-Wasser, 93°C, 5 min,
- Trocknung.

Die so behandelten Instrumente werden auf der sog. reinen Seite der zentralen Instrumentenaufbereitung 3 überprüft, ggf. mit Pflegemitteln behandelt und zu Instrumentensets verpackt. Anschließend erfolgt eine übliche Dampfsterilisation.

Die sterilisierten Instrumentensets werden in das Sterillager 4 verbracht und dort für die nächste Verwendung bereit gehalten.

## Patentansprüche

1. Verfahren zur Aufbereitung medizinischer und/oder chirurgischer Instrumente und Apparate, mit den Schritten:
a) Einlegen der gebrauchten Instrumente und/oder Apparate in eine Instrumentenspülmaschine, vorzugsweise in eine Eintank-Instrumentenspülmaschine;
b) Durchführen einer maschinellen Prädekontamination;
c) Entnehmen der Instrumente und/oder Apparate aus der Instrumentenspülmaschine;
d) Überführen der Instrumente und/oder Apparate zu einer zentralen Instrumentenaufbereitungsstation;
e) Durchführen einer vollständigen Reinigung, Desinfektion und Sterilisation der Instrumente und/oder Apparate in der zentralen Instrumentenaufbereitungsstation zur Wiederherstellung eines gebrauchsfertigen Zustands der Instrumente und/oder Apparate.

2. Verfahren nach Anspruch 1, **dadurch gekennzeichnet, dass** die Instrumentenspülmaschine im Operationsbereich angeordnet ist.

3. Verfahren nach Anspruch 1 oder 2, **dadurch gekennzeichnet, dass** die maschinelle Prädekontamination eine Reinigung mit einem tensidhaltigen Reinigungsmittel, das anwendungsfertig verdünnt in wässriger Lösung einen pH-Wert von wenigstens 10,5 aufweist, umfasst.

4. Verfahren nach Anspruch 3, **dadurch gekennzeichnet, dass** die maschinelle Prädekontamination folgende Schritte umfasst:
f) Vorspülen;
g) Reinigen mit einem tensidhaltigen Reinigungsmittel, das anwendungsfertig verdünnt in wässriger Lösung einen pH-Wert von wenigstens 10,5 aufweist;
h) Neutralisieren und/oder Spülen.

5. Verfahren nach Anspruch 3 oder 4, **dadurch gekennzeichnet, dass** das tensidhaltige Reinigungsmittel Alkalihydroxide, vorzugsweise Kaliumhydroxid, enthält und dass vorzugsweise in der anwendungsfertig verdünnten Lösung der Alkalihydroxidgehalt 200 bis 10.000 ppm, weiter vorzugsweise 200 bis 5.000 ppm, weiter vorzugsweise 200 bis 2.000 ppm beträgt.

6. Verfahren nach einem der Ansprüche 3 bis 5, **dadurch gekennzeichnet, dass** die Einwirkzeit des Reinigungsmittels 1 bis 30 min, vorzugsweise 2 bis 20 min, weiter vorzugsweise 3 bis 15 min, weiter vorzugsweise 5 bis 10 min beträgt.

7. Verfahren nach einem der Ansprüche 3 bis 6, **dadurch gekennzeichnet, dass** die Reinigung bei einer Temperatur von Raumtemperatur bis 93°C, vorzugsweise 40 bis 93°C, weiter vorzugsweise 50 bis 80°C, weiter vorzugsweise 50 bis 60°C stattfindet.

8. Verfahren nach einem der Ansprüche 3 bis 7, **dadurch gekennzeichnet, dass** das tensidhaltige Reinigungsmittel anwendungsfertig verdünnt in wässriger Lösung einen pH-Wert von wenigstens 11, vorzugsweise wenigstens 11,5, vorzugsweise wenigstens 12, weiter vorzugsweise wenigstens 12,5 aufweist.

9. Verfahren nach einem der Ansprüche 3 bis 8, **dadurch gekennzeichnet, dass** das Reinigungsmittel kationische, nichtionische und/oder amphotere Tenside enthält.

10. Verfahren nach einem der Ansprüche 3 bis 9, **dadurch gekennzeichnet, dass** in dem Reinigungsmittel in der anwendungsfertig verdünnten Lösung der Gehalt an kationischen Tensiden 15 bis 500 ppm, vorzugsweise 15 bis 100 ppm, weiter vorzugsweise 15 bis 50 ppm beträgt.

11. Verfahren nach einem der Ansprüche 3 bis 10, **dadurch gekennzeichnet, dass** das Reinigungsmittel einen pH-Wert von wenigstens 11 in anwendungsfertig verdünnter Reinigerlösung aufweist, und quarternäre Ammoniumverbindungen (QAV) sowie amphotere Tensiden enthält.

12. Verfahren nach Anspruch 11, **dadurch gekennzeichnet, dass** das Gewichtsverhältnis von amphoteren Tensiden zu QAV in der Reinigerlösung 2,3:1 bis 5:1, weiter vorzugsweise 2,3:1 bis 4:1, besonders bevorzugt 2,3:1 bis 3:1 beträgt und Konzentration der QAV in der verdünnten Reinigerlösung zwischen 15 und 100 ppm, vorzugsweise 15 und 50 ppm, weiter vorzugsweise 15 und 30 ppm liegt.

13. Verfahren nach einem der Ansprüche 3 bis 12, **dadurch gekennzeichnet, dass** in dem Reinigungsmittel in der anwendungsfertig verdünnten Lösung der Gehalt an amphoteren Tensiden 50 bis 1.000 ppm, vorzugsweise 100 bis 500 ppm, weiter vorzugsweise 150 bis 300 ppm beträgt.

14. Verfahren nach einem der Ansprüche 3 bis 13, **dadurch gekennzeichnet, dass** das Reinigungsmittel anwendungsfertig verdünnt eine Oberflächenspannung von weniger als 50 mN/m, vorzugsweise weniger als 40 mN/m, weiter vorzugsweise weniger als 35 mN/m aufweist.

15. Verfahren nach einem der Ansprüche 1 bis 14, **dadurch gekennzeichnet, dass** das Überführen der vorgereinigten Instrumente und/oder Apparate zu einer zentralen Instrumentenaufbereitungsstation trocken erfolgt.

## Claims

1. A method of treating medical and/or surgical instruments and apparatus, with the steps of:
a) placing the used instruments and/or apparatus in an instrument-rinsing machine, preferably in a single-tank instrument-rinsing machine,
b) carrying out a mechanical pre-decontamination;
c) removing the instruments and apparatus from the instrument-rinsing machine;
d) transferring the instruments and/or apparatus to a central instrument-treatment station;
e) carrying out complete cleansing, disinfection and sterilisation of the instruments and/or apparatus in the central instrument-treatment station to re-establish a ready-for-use condition of the instruments and/or apparatus.

2. A method according to Claim 1, **characterised in that** the instrument-rinsing machine is disposed in the operating zone.

3. A method according to Claim 1 or 2, **characterised in that** the mechanical pre-decontamination comprises cleansing with a surfactant-containing cleansing agent which, diluted ready for use in an aqueous solution, has a pH value of at least 10.5.

4. A method according to Claim 3, **characterised in that** the mechanical pre-decontamination comprises the following steps:
f) prerinsing;
g) cleansing with a surfactant-containing cleansing agent which, diluted ready for use in an aqueous solution, has a pH value of at least 10.5;
h) neutralising and/or rinsing.

5. A method according to Claim 3 or 4, **characterised in that** the surfactant-containing cleansing agent contains alkali hydroxides, preferably potassium hydroxide, and **in that** preferably in the diluted ready-for-use solution the alkali hydroxide content amounts to 200 to 10,000 ppm, more preferably 200 to 5,000 ppm, more preferably 200 to 2,000 ppm.

6. A method according to any one of Claims 3 to 5, **characterised in that** the action time of the cleansing agent amounts to 1 to 30 min., preferably 2 to 20 min., more preferably 3 to 15 min., more preferably 5 to 10 min.

7. A method according to any one of Claims 3 to 6, **characterised in that** the cleansing takes place at a temperature of from room temperature to 93°C, preferably 40 to 93°C, more preferably 50 to 80°C, more preferably 50 to 60°C.

8. A method according to any one of Claims 3 to 7, **characterised in that** the surfactant-containing cleansing agent diluted ready-for-use in aqueous solution has a pH value of at least 11, preferably at least 11.5, preferably at least 12, more preferably at least 12.5.

9. A method according to any one of Claims 3 to 8, **characterised in that** the cleansing agent contains cationic, non-ionic and/or amphoteric surfactants.

10. A method according to any one of Claims 3 to 9, **characterised in that** in the cleansing agent in the diluted ready-for-use solution the content of cationic surfactants amounts to 15 to 500 ppm, preferably 15 to 100 ppm, more preferably 15 to 50 ppm.

11. A method according to any one of Claims 3 to 10, **characterised in that** the cleansing agent has a pH value of at least 11 in the diluted ready-for-use cleansing agent solution, and contains quaternary ammonium compounds (QAC) as well as amphoteric surfactants.

12. A method according to Claim 11, **characterised in that** the weight ratio of amphoteric surfactants to QAC in the cleansing agent solution amounts to 2.3:1 to 5:1, more preferably 2.3:1 to 4:1, especially preferably 2.3:1 to 3:1, and the concentration of the QAC in the diluted cleansing agent solution is between 15 and 100 ppm, preferably 15 and 50 ppm, more preferably 15 and 30 ppm.

13. A method according to any one of Claims 3 to 12, **characterised in that** in the cleansing agent in the diluted ready-for-use solution the content of amphoteric surfactants amounts to 50 to 1,000 ppm, preferably 100 to 500 ppm, more preferably 150 to 300 ppm.

14. A method according to any one of Claims 3 to 13, **characterised in that** the cleansing agent, diluted ready-for-use, has a surface tension of lower than 50 mN/m, preferably lower than 40 mN/m, more preferably lower than 35 mN/m.

15. A method according to any one of Claims 1 to 14, **characterised in that** the transfer of the precleansed instruments and/or apparatus to a central instrument-treatment station takes place in a dry state.

## Revendications

1. Procédé de traitement d'instruments et appareils médicaux et/ou chirurgicaux, comprenant les étapes suivantes:
a) insertion des instruments et/ou appareils usagés dans une machine de rinçage d'instruments, de préférence dans une machine de rinçage d'instruments monocuve;
b) exécution d'une pré-décontamination mécanique;
c) enlèvement des instruments et/ou appareils de la machine de rinçage d'instruments;
d) transfert des instruments et/ou appareils vers une station centrale de traitement d'instruments;
e) exécution d'un nettoyage complet, d'une désinfection et d'une stérilisation des instruments et/ou appareils dans la station centrale de traitement d'instruments, afin de rétablir un état prêt à l'utilisation des instruments et/ou appareils.

2. Procédé selon la revendication 1, **caractérisé en ce que** la machine de rinçage d'instruments est disposée dans la zone d'opération.

3. Procédé selon la revendication 1 ou 2, **caractérisé en ce que** la pré-décontamination mécanique comprend un nettoyage avec un produit de nettoyage contenant des tensioactifs qui, dilué prêt à l'utilisation dans une solution aqueuse, présente un pH d'au moins 10,5.

4. Procédé selon la revendication 3, **caractérisé en ce que** la pré-décontamination mécanique comprend les étapes suivantes:
f) pré-rinçage;
g) nettoyage avec un produit de nettoyage contenant des tensioactifs qui, dilué prêt à l'utilisation dans une solution aqueuse, présente un pH d'au moins 10,5;
h) neutralisation et/ou rinçage.

5. Procédé selon la revendication 3 ou 4, **caractérisé en ce que** le produit de nettoyage contenant des tensioactifs contient des hydroxydes alcalins, de préférence de l'hydroxyde de potassium, et **en ce que** la teneur en hydroxydes alcalins dans la solution diluée prête à l'utilisation est de préférence comprise entre 200 et 10.000 ppm, mieux entre 200 et 5.000 ppm, mieux encore entre 200 et 2.000 ppm.

6. Procédé selon l'une quelconque des revendications 3 à 5, **caractérisé en ce que** le temps d'action du produit de nettoyage est de 1 à 30 min., de préférence de 2 à 20 min., mieux de 3 à 15 min., mieux encore de 5 à 10 min.

7. Procédé selon l'une quelconque des revendications 3 à 6, **caractérisé en ce que** le nettoyage s'effectue à une température allant de la température ambiante à 93°C, de préférence de 40 à 93°C, mieux de 50 à 80°C, mieux encore de 50 à 60°C.

8. Procédé selon l'une quelconque des revendications 3 à 7, **caractérisé en ce que** le produit de nettoyage contenant des tensioactifs, dilué prêt à l'utilisation dans une solution aqueuse, présente un pH d'au moins 11, de préférence d'au moins 11,5, mieux d'au moins 12, mieux encore d'au moins 12,5.

9. Procédé selon l'une quelconque des revendications 3 à 8, **caractérisé en ce que** le produit de nettoyage contient des tensioactifs cationiques, non ioniques et/ou amphotères.

10. Procédé selon l'une quelconque des revendications 3 à 9, **caractérisé en ce que** la teneur en tensioactifs cationiques du produit de nettoyage dans la solution diluée prête à l'utilisation est comprise entre 15 et 500 ppm, de préférence entre 15 et 100 ppm, mieux entre 15 et 50 ppm.

11. Procédé selon l'une quelconque des revendications 3 à 10, **caractérisé en ce que** le produit de nettoyage présente un pH d'au moins 11 en solution de nettoyant diluée prête à l'utilisation, et contient des composés d'ammonium quaternaire (QAV) ainsi que des tensioactifs amphotères.

12. Procédé selon la revendication 11, **caractérisé en ce que** le rapport de poids des tensioactifs amphotères aux QAC dans la solution de nettoyant est de 2,3:1 à 5:1, d'une manière plus préférée de 2,3:1 à 4:1, d'une manière particulièrement préférée de 2,3:1 à 3:1, et la concentration de QAV dans la solution de nettoyant diluée est comprise entre 15 et 100 ppm, de préférence entre 15 et 50 ppm, mieux entre 15 et 30 ppm.

13. Procédé selon l'une quelconque des revendications 3 à 12, **caractérisé en ce que** la teneur en tensioactifs amphotères du produit de nettoyage dans la solution diluée prête à l'utilisation est comprise entre 50 et 1.000 ppm, de préférence entre 100 et 500 ppm, mieux entre 150 et 300 ppm.

14. Procédé selon l'une quelconque des revendications 3 à 13, **caractérisé en ce que** le produit de nettoyage, dilué prêt à l'utilisation, présente une tension superficielle inférieure à 50 mN/m, de préférence inférieure à 40 mN/m, mieux inférieure à 35 mN/m.

15. Procédé selon l'une quelconque des revendications 1 à 14, **caractérisé en ce que** le transfert des instruments et/ou appareils pré-nettoyés vers une station centrale de traitement d'instruments s'effectue à sec.
